**Europäisches Patentamt**

**European Patent Office**

**Office européen des brevets**

(19)

(11) Numéro de publication: **0 072 513
B1**

# FASCICULE DE BREVET EUROPÉEN

(12)

(45) Date de publication du fascicule du brevet:
**29.03.89**

(21) Numéro de dépôt: **82107154.5**

(22) Date de dépôt: **07.08.82**

(51) Int. Cl.⁴: **A 61 K 39/095,** A 61 K 39/102, C 12 P 19/00, A 61 K 39/09 // C12R1/21, C12R1/36, C12R1/46

(54) **Procédé de préparation de polysaccharides bactériens capsulaires antigéniques purifiés, produits obtenus et leur utilisation.**

(30) Priorité: **14.08.81 BE 205681**

(43) Date de publication de la demande:
**23.02.83 Bulletin 83/8**

(45) Mention de la délivrance du brevet:
**29.03.89 Bulletin 89/13**

(84) Etats contractants désignés:
**AT CH DE FR GB IT LI LU NL SE**

(56) Documents cité:
**EP-A-0 002 404
EP-A-0 017 322
EP-A-0 038 265
FR-A-2 388 563
FR-A-2 406 999
FR-A-2 407 000
US-A-3 636 192
US-A-3 978 209
US-A-4 123 520
US-A-4 134 214
US-A-4 235 994
US-A-4 242 501
US-A-4 264 764
US-A-4 407 949**

(73) Titulaire: **Smith Kline - RIT Société anonyme dite:, rue du Tilleul, 13, B-1320 Rixensart (Genval) (BE)**

(72) Inventeur: **Liveyns, Robert W., Degreefstraat, 3b, B-1990 Hoeilaart (BE)**
Inventeur: **Gilles, Daniel, Rue Mascau, 13, B-1320 Genval (BE)**
Inventeur: **Arickx, Marcel, Chaussée de Tirlemont, 13, B-5906 Saint- Jean Geest (BE)**

(74) Mandataire: **Tasset, Gérard, SMITHKLINE - RIT rue de l'Institut, 89, B-1330 Rixensart (BE)**

## Description

La présente invention est relative à un procédé de préparation de polysaccharides bactériens purifiés, aux produits ainsi obtenus et à leur utilisation.

Certaines bactéries doivent leur virulence à la présence d'une capsule qui enveloppe le germe et la plupart de ces capsules - c'est notamment le cas des pneumocoques, des méningocoques et de Haemophilus influenzae - sont notamment composées de polysaccharides de hauts poids moléculaires.

Cette capsule polysaccharidique sert d'"armure" au germe, empêchant ainsi sa phagocytose par les neutrophiles polymorphonucléaires et, pour l'organisme hôte, cette capsule nécessite des moyens de défense plus élaborés.

C'est également la nature des polysaccharides constituant la capsule qui permet le typage ou le groupage de la bactérie chez les pneumocoques, méningocoques ou chez Haemophilus influenzae.

Ainsi par exemple, on a jusqu'ici identifié et typé 83 souches de Streptococcus pneumoniae, le typage se réalisant en mettant une bactérie vivante en contact avec un antisérum spécifique. Dans ce cas, la capsule polysaccharidique responsable de la virulence du germe réagit avec les anticorps, provoquant un gonflement de la capsule et celui-ci est observable au microscope.

Parmi les 83 souches identifiées jusqu'ici, une quarantaine sont couramment isolées de cas cliniques de pneumonie, de méningite et d'otite et on estime que, suivant la région du globe, environ 12 à 25 souches sont respectivement responsables d'environ 70 à 90 % des infections à Streptococcus pneumoniae.

Ces différentes souches ont été codifiées suivant deux nomenclatures légèrement différentes l'une de l'autre, à savoir la nomenclature danoise et la nomenclature américaine. Dans la description et les revendications qui suivent, c'est la nomenclature danoise qui est couramment utilisée. Les principales souches ont également fait l'objet d'un dépôt à l'American Type Culture Collection (ATCC), Rockville, Maryland, U.S.A. où elles sont librement disponibles, les autres souches pouvant être isolées de cas cliniques ou obtenues par exemple au Statens Seruminstitut à Copenhague, Danemark.

Le tableau I suivant donne, à titre indicatif, les principaux sérotypes de Streptococcus pneumoniae avec leur nomenclature américaine, danoise et ATCC.

## Nomenclature

| américaine | danoise | ATCC |
|---|---|---|
| 1 | 1 | 6301 |
| 2 | 2 | 6302 |
| 3 | 3 | 6303 |
| 4 | 4 | 6304 |
| 6 | 6 A | 6306 |
| 8 | 8 | 6308 |
| 9 | 9 N | 6309 |
| 12 | 12 F | 6312 |
| 14 | 14 | 6314 |
| 15 | 15 F | 6315 |
| 17 | 17 F | 6317 |
| 19 | 19 F | 6319 |
| 20 | 20 | 6320 |
| 23 | 23 F | 6323 |
| 25 | 25 | 6325 |
| 26 | 6 B | 6326 |
| 51 | 7 F | 10351 |
| 56 | 18 C | 10356 |
| 57 | 19 A | 10357 |

La culture des pneumocoques, des méningocoques et H. influenzae type b en laboratoire et l'isolement de leurs polysaccharides capsulaires sont connus depuis longtemps mais la qualité des produits isolés dépend dans une certaine mesure des méthodes de culture, d'extraction et de purification, ces dernières étant généralement basées sur des techniques de précipitations successives à l'alcool, de formation de complexe avec le bromure de cétrimonium (Cetavlon) et/ou de traitement enzymatique, le but final de ces opérations étant de séparer le polysaccharide de divers composés polyioniques tels que, suivant le cas, protéines, acides nucléiques, lipopolysaccharides et, éventuellement, polysaccharides "parasites" comme la substance "C" de pneumocoques.

Une revue de polysaccharides pneumococciques a été publiée par exemple dans Adv. Carbohydr. Chem. and Biochem. 33, p. 295 - 322, 1976.

Des polysaccharides ainsi isolés peuvent être utilisés par exemple dans la prophylaxie de la pneumonie et des vaccins contenant un certain nombre de ces polysaccharides sont connus par exemple par la demande de brevet européen publiée sous le No 2 404 et par le brevet No 4 242 501 des Etats-Unis d'Amérique.

Le méningocoque ou Neisseria meningitidis est également différencié en divers sérogroupes (A, B, C, D, E, X, Y, Z, 29 e et W-135) suivant la nature des polysaccharides de la capsule, les principaux sérogroupes étant les sérogroupes A, B et C.

Le polysaccharide de N. meningitidis, sérogroupe A (ou PS-A) est une substance bien caractérisée: il s'agit d'un polymère de sucre aminé et il a été identifié comme étant formé d'unités de phosphate de N-acétylmannosamine partiellement O-acétylée.

Le polysaccharide de N. meningitidis, sérogroupe C (ou PS-C) est également un polymère de sucre aminé; il a été identifié comme étant formé d'unités d'acide N-acétylneuraminique partiellement mono- et di- O-acétylé.

Le polysaccharide C⁻ correspond au polysaccharide C non-acétylé.

Le polysaccharide de N. meningitidis, sérogroupe W-135 (ou PS-W) est également une substance bien caractérisée: il s'agit d'un copolymère partiellement O-acétylé formé d'unités d'acide N-acétylneuraminique et de galactose.

Le polysaccharide de N. meningitidis, sérogroupe Y (ou PS-Y) est un copolymère partiellement O-acétylé formé d'unités d'acide N-acétylneuraminique et de glucose.

Le polysaccharide de H. influenzae type b (ou PRR'P) est une substance bien caractérisée: il s'agit d'un polymère de phosphate de ribosylribitol.

L'activité immunogène de ces polysaccharides est directement liée au degré de polymérisation, toute dépolymérisation substantielle pouvant se produire par exemple en cours d'extraction, de purification ou de stockage entraînant une forte chute de l'activité immunogène.

Comme les sérogroupes A et C, le sérogroupe B de Neisseria meningitidis présente une diversité antigénique connue d'ailleurs depuis longtemps. La partie externe de ce microorganisme est composée de polysaccharides capsulaires, lipopolysaccharides (LPS), protéines et lipides et les trois antigènes (polysaccharides, LPS et protéines) semblent être importants dans l'immunité acquise après infection naturelle chez l'homme. Les polysaccharides B et C sont des homopolymères acétylés de l'acide neuraminique mais des différences importantes entre ces deux polysaccharides apparaissent lors d'une analyse plus fine des structures: le polysaccharide B ne possède en effet pas de groupement O-acétyle et l'enchaînement osidique est alpha 2 - 8, alors que le polysaccharide C est partiellement O-acétylé et la liaison osidique est alpha 2 - 9.

Le brevet 3 636 192 des Etats-Unis d'Amérique décrit un vaccin antiméningococcique contenant les polysaccharides méningococciques des groupes A et C et leur préparation au départ de souches connues de Neisseria meningitidis (groupe A et groupe C).

Le polysaccharide capsulaire de Haemophilus influenzae type b (PRR'P) est un produit connu dont l'isolement, la purification et l'usage sont décrits par exemple dans le brevet 4 220 717 des Etats-Unis d'Amérique.

La présente invention concerne un procédé relativement simple pour la préparation de polysaccharides antigéniques capsulaires, notamment de pneumocoques, de méningocoques et de Haemophilus influenzae remarquablement débarrassés de contaminants tels que cités plus haut, c'est-à-dire notamment des protéines, acides nucléiques et, éventuellement et suivant le cas, des lipopolysaccharides ou de la substance C (jusqu'à moins de 0,2 %). Les produits ainsi obtenus sont apyrogènes à la dose de 5 µg par ml et par kg de lapin (méningocoques) et différents essais (pneumocoques) ont montré que leur administration n'induit pas une augmentation d'anticorps isohémagglutinine A chez les vaccinés de groupe sanguin O ou B, évitant ainsi les risques d'induction de maladie hémolytique chez les nouveaux-nés de mères vaccinées.

De plus, leur lyophilisation et leur stockage ne requièrent pas les procédés ou additifs spéciaux qui font par exemple l'objet du brevet No 4 134 214 des Etats-Unis d'Amérique, du brevet belge No 871 612 et de la demande de brevet européen publiée sous le numéro 17 322.

Le procédé de l'invention est un procédé d'extraction et/ou de purification d'un polysaccharide bactérien capsulaire antigénique comprenant au moins un stade de formation de complexe entre au moins un dérivé polyionique du milieu et un sel d'ammonium quaternaire (comme, par exemple, le bromure de cétrimonium (Cetavlon) ou le bromure de cétylpyridinium), caractérisé en ce que au moins une - et de préférence chaque - précipitation de complexe est effectuée sur un support poreux inerte.

Suivant une particularité de l'invention, on procède à une séparation d'un ou de plusieurs dérivés polyioniques du milieu sur la base de la nature du sel et de la concentration saline spécifique qui déterminent la formation ou la décomposition du complexe de ce dérivé polyionique ou de chacun de ces dérivés polyioniques avec un sel d'ammonium quaternaire comme le Cetavlon.

Dans le procédé de l'invention, le support poreux inerte peut être par exemple de la cellulose, un sel alcalinoterreux substantiellement insoluble dans l'eau, un oxyde d'aluminium comme, par exemple, la bauxite ou l'alumine, un hydroxyde d'aluminium, un oxyde de silicium ou un silicate comme, par exemple, le silicagel, les terres d'infusoires ou diatomées, la terre de Fuller et autres dérivés mixtes comme par exemple les aluminosilicates, ces exemples n'étant pas limitatifs, le but du support poreux - qui peut se présenter par exemple sous forme poudreuse, granuleuse ou fibreuse - étant d'éviter la formation d'un précipité gommeux et de favoriser une redissolution sélective. Un exemple de support poreux inerte particulièrement intéressant est constitué par du kieselguhr® comme la Celite 545® (une terre de diatomées vendue par Johns-Manville, Denver, Colo. U.S.A.).

Etant donné que les polysaccharides capsulaires sont soit des dérivés polyioniques soit des dérivés sensiblement neutres (ces derniers ne formant pas de complexe avec les sels d'ammonium quaternaire) mais que les impuretés sont généralement de nature polyionique, il est évident que le procédé de l'invention permet d'obtenir sous forme purifiée aussi bien un polysaccharide polyionique qu'un polysaccharide neutre, le principe du procédé étant basé sur la spécificité des concentrations salines qui règlent la formation ou la décomposition des complexes entre un sel d'ammonium quaternaire et différents composés polyioniques.

Le procédé de l'invention est indistinctement applicable aux moûts de fermentation et aux solutions aqueuses de polysaccharides capsulaires bruts.

Une forme particulièrement intéressante de réalisation de l'invention consiste à appliquer le procédé à un moût de fermentation pour lequel on aura utilisé un milieu synthétique ou semi-synthétique tel que décrit par exemple par Mark A. ADAMS et Amy S. ROE dans J. Bact. 49 p. 401 - 409, 1945 et par Paul D. HOEPRICH dans J. Bact. 69, p. 682 - 688, 1955 et J. Bact. 74, p. 587 - 590, 1957 ou tout autre milieu du même genre pour la culture de Streptococcus pneumoniae.

Des milieux similaires pour la culture de Neisseria meningitidis et de Haemophilus influenzae sont par exemple respectivement les milieux décrits par I.D. FRANTZ dans J. Bact. 43, 757 - 761, 1942 et par E.C. GOTSCHLICH et al. dans Progr. immunobiol. Standard, vol. 5, 485 - 491, 1972 d'une part et par P. ANDERSON et al. dans Infect. and Immun. 13, 581 - 589, 1976 d'autre part.

Dans le cas de la préparation d'un polysaccharide méningococcique du groupe A, B, C, C-, Y ou W-135 ou de H. influenzae type b au départ d'un milieu de culture, on procède plus particulièrement en suivant le schéma suivant:
(a) isolement du polysaccharide par précipitation sous forme de complexe avec le sel d'ammonium quaternaire (par exemple Cetavlon®), en présence d'un support inerte (par exemple de la Celite 545);
(b) traitement du précipité par une solution ionique aqueuse ou aqueuse/éthanol (jusqu'à 60 % v/v en éthanol) de telle sorte que la concentration finale soit comprise entre 0,1 et 0,6 équivalent-gramme de sel (par exemple chlorure de sodium, de calcium ou d'ammonium) par litre et récolte de la solution;
(c) précipitation du polysaccharide par addition subséquente d'éthanol;
(d) récolte du précipité et dissolution dans une solution ionique aqueuse, de préférence du chlorure de calcium à une concentration comprise entre 0,1 et 2 N;
(e) dialyse de la solution;
(f) précipitation du polysaccharide par addition d'éthanol.

Dans le cas de la préparation d'un polysaccharide pneumococcique anionique au départ d'un milieu de culture (de préférence synthétique), on procédera plus particulièrement en suivant le schéma suivant:
(a) séparation d'une partie des composants polyioniques du milieu par précipitation sous forme de complexes avec le sel d'ammonium quaternaire (par exemple Cetavlon), en présence d'un support poreux inerte et à une concentration ionique de milieu comprise entre 0,15 N et 1 N en sel.
(b) récolte de la solution de polysaccharide, par filtration;
(c) concentration du milieu et ajustement de sa concentration ionique à la valeur à laquelle se forme le complexe insoluble entre le polysaccharide et le sel d'ammonium quaternaire (par exemple Cetavlon), en présence d'un support poreux inerte à pH 5 - 5,2;
(d) récolte du précipité par filtration;
(e) dissolution en milieu aqueux ionique au moins 0,10 N en sel (de préférence entre 0,1 et 1 N en acétate de sodium);
(f) clarification;
(g) précipitation par addition d'éthanol.

Dans le cas de la préparation d'un polysaccharide pneumococcique neutre au départ d'un milieu de culture (de préférence synthétique), on procède plus particulièrement suivant le schéma suivant:
(a) séparation d'une partie des composants polyioniques du milieu par précipitation sous forme de complexes avec le sel d'ammonium quaternaire (par exemple Cetavlon), en présence d'un support poreux inerte;
(b) récolte de la solution du polysaccharide, par filtration;
(c) concentration;
(d) précipitation du polysaccharide par addition de 3 volumes d'éthanol;
(e) dissolution du précipité dans une solution tampon cationique 0,005 N à pH 7 ( ± 0,1) et traitement à la diéthylaminoéthyl cellulose;
(f) clarification;
(g) précipitation du polysaccharide par addition de 3 volumes d'éthanol;

Dans les exemples qui suivent et qui ne sont d'ailleurs pas limitatifs de l'invention, le milieu de culture utilisé pour les souches de Neisseria meningitidis est le milieu décrit par E.C. GOTSCHLICH et al. dans Progr. immunol. Standard., vol. 5, 485 - 491, 1972, le milieu de culture utilisé pour Haemophilus influenzae est le milieu décrit par P. ANDERSON et al. dans Infect. and Immun. 13, 581 - 589, 1976 et le milieu de culture utilisé pour les souches de Streptococcus pneumoniae est celui qui est décrit par Paul D. HOEPRICH (loc. cit.).

Des vaccins monovalents ou polyvalents peuvent être aisément préparés avec les polysaccharides capsulaires purifiés de la présente invention. A cette fin, comme il est bien connu dans la pratique pour la préparation de tels vaccins, les polysaccharides sont par exemple dissous dans de l'eau ou dans une solution saline contenant éventuellement un stabilisant et/ou un antiseptique et l'ensemble est soumis à une filtration stérilisante, mis en flacons et, éventuellement, lyophilisé.

## Exemple 1

On prépare une culture de Neisseria meningitidis groupe A suivant les indications du brevet No 3 636 192 de Etats-Unis d'Amérique et le moût obtenu à la fin du cycle de fermentation est inactivé par chauffage pendant 10 minutes à 56° C et centrifugé à 10.000 g dans une centrifugeuse Sharpless de type AS 16 pour donner 80 litres de jus de culture inactivé.

On ajoute sous agitation 2.400 g de Celite 545 (un produit vendu par Johns-Manville, Denver, Colo., U.S.A.) et on transfère la suspension dans une cuve de 140 litres refroidie à l'eau glacée. Sous forte agitation, on ajoute 1 600 ml d'une solution à 5 % (p/v) de Cetavlon (un produit vendu par Aldrich Europe, Beerse, Begique) dans de l'eau désionisée et on maintient l'agitation pendant 10 minutes après addition complète du Cetavlon.

On laisse ensuite reposer pendant une heure à 5° C pour permettre la décantation du complexe polysaccharide/ Cetavlon et de la Celite 545. Le surnageant est éliminé après décantation et le précipité est filtré sous pression réduite (20 mm de mercure) et lavé chromatographiquement sur le filtre avec une solution aqueuse de Cetavlon (0,05 % p/v). Le gâteau est essoré au maximum et la quantité d'eau résiduelle déterminée par pesée s'élève à 1,4 l.

Par agitation mécanique pendant 10 minutes, ce gâteau est dispersé dans un mélange de 4,5 l d'éthanol et de 2,3 l d'eau dans laquelle on dissout 216,5 g de chlorure de sodium (de manière à obtenir une composition finale de l'éluant 0,45 N en chlorure de sodium dans un mélange eau/éthanol 45/55).

Le milieu est ensuite filtré et on recueille le filtrat. Le gâteau est élué chromatographiquement avec 8,2 litres du même mélange 0,45 N en chlorure de sodium dans le mélange eau/éthanol 45/55.

Sous agitation, on ajoute de l'éthanol aux filtrats réunis pour obtenir une concentration finale de 80 % en éthanol et on laisse au repos à 40° C pendant 24 heures de manière à permettre la précipitation du polysaccharide brut. On élimine le surnageant par décantation et on triture le précipité avec 3 litres d'éthanol. On filtre, on lave à l'éthanol et à l'acétone et on sèche sous pression réduite (20 mm de mercure) à température ordinaire pendant 15 heures. On obtient ainsi 12 g de polysaccharide groupe A brut.

Le polysaccharide brut est dissous dans 4,8 l d'une solution 0,2 N de chlorure de calcium. La solution est ultrafiltrée à 2,5 litres sur un appareil AMICON DC 10 muni d'une cartouche de cut-off 10.000 et, après diafiltration en continu avec 15 litres d'une solution 0,2 N de chlorure de calcium, le volume est réduit à environ 2 litres et le dialyseur est rincé avec une solution 0,2 N de chlorure de calcium pour obtenir un volume final d'environ 4 litres.

Le rétentat est précipité par addition de 4 volumes d'éthanol et, après un repos de plusieurs heures à 40° C, le précipité est filtré, lavé à l'éthanol et à l'acétone et séché sous pression réduite (20 mm de mercure) à température ambiante pendant 15 heures pour donner du polysaccharide A purifié.

## Exemple 2

La technique est celle de l'exemple 1 mais on remplace la Celite 545 par de la Celite 512 ou par de la Celite 560 (Celite 512 et Celite 560 sont des terres de diatomées vendues par Johns-Manville, Denver, Colo. U.S.A.) et on obtient ainsi dans chaque cas un produit correspondant sensiblement au produit de l'exemple 1.

## Exemple 3

La technique est celle de l'exemple 1 mais on remplace la Celite 545 par de la Perlite SH® (un aluminosilicate vendu par Dicalite Europe Nord, Bruxelles, Belgique) et la composition finale de l'éluant est 0,3 N en chlorure de calcium dans un mélange eau/éthanol 50/50. On obtient ainsi finalement un produit correspondant sensiblement au produit de l'exemple 1.

## Exemple 4

La technique est celle de l'exemple 1 mais on procède au départ d'une culture de Neisseria meningitidis groupe C et on précipite par addition de 3 volumes d'éthanol. On obtient ainsi du polysaccharide groupe C purifié.

## Exemple 5

La technique est celle de l'exemple 1 mais on procède au départ d'une culture de Neisseria meningitidis groupe B et la composition finale de l'éluant est 0,21 N en chlorure de sodium dans un mélange eau/éthanol 60/40. On obtient ainsi du polysaccharide groupe B purifié.

## Exemple 6

La technique est celle de l'exemple 1 mais on remplacera la celite 545 par de la Perlite 4158 (un aluminosilicate vendu par Dicalite Europe Nord, Bruxelles, Belgique), le lavage est effectué avec une solution aqueuse 0,05 N de chlorure d'ammonium et la composition finale de l'éluant est 0,25 N en chlorure d'ammonium dans de l'eau.

On obtient ainsi du polysaccharide groupe W-135 purifié.

## Exemple 7

La technique est celle de l'exemple 1 mais on procède au départ d'une culture de Neisseria meningitidis groupe Y, la composition finale de l'éluant est 0,12 N en chlorure de sodium dans un mélange eau/éthanol 52/48 et on précipite par addition de 3 volumes d'éthanol. On obtient ainsi du polysaccharide groupe Y purifié.

## Exemple 8

La technique est celle de l'exemple 1 mais on procède au départ d'une culture de Haemophilus influenzae type b, la composition finale de l'éluant est 0,21 N en chlorure de sodium dans un mélange eau/éthanol 60/40 et on précipite par addition de 3 volumes d'éthanol à 80 %. On obtient ainsi du polysaccharide de Haemophilus influenzae type b purifié.

## Exemple 9

La technique est celle de l'exemple 4 mais en remplace la Celite 545 respectivement par de la poudre de cellulose naturelle (une poudre de cellulose vendue par E. Merck, Darmstadt, R.F.A.), de la Perlite 4158 (un aluminosilicate vendu par Dicalite Europe Nord, Bruxelles, Belgique), du Hyflo Supercel® (une terre de diatomées vendue par Johns-Manville, Denver, Colo., U.S.A.), du Hysorb V®$_{100}$ 100 (une terre de diatomées vendue par Johns-Manville, Denver, Colo., U.S.A.) et du Filter Cel®(une terre de diatomées vendue par Johns-Manville, Denver, Colo., U.S.A.).

On obtient ainsi dans chaque bas du polysaccharide C purifié correspondant sensiblement au produit de l'exemple 4.

## Exemple 10

La technique est celle de l'exemple 4 mais la composition finale de l'éluant est 0,3 N en chlorure de sodium dans un mélange eau/éthanol 50/50.

On obtient ainsi finalement du polysaccharide C purifié correspondant sensiblement au produit de l'exemple 4.

## Exemple 11

La technique est celle de l'exemple 10 mais on remplace le lavage avec la solution de Cetavlon à 0,05 % par un lavage à l'eau et la composition finale de l'éluant est 0,15 N en chlorure de sodium dans un mélange eau/éthanol 50/50.

On obtient ainsi finalement du polysaccharide C purifié correspondant sensiblement au produit de l'exemple 4.

## Exemple 12

La technique est celle de l'exemple 4 mais la composition finale de l'éluant est 0,135 N en chlorure de sodium dans un mélange eau/éthanol 45/55.

On obtient ainsi finalement du polysaccharide C purifié correspondant sensiblement au produit de l'exemple 4.

## Exemple 13

La technique est celle de l'exemple 10 mais on remplace le chlorure de sodium par de l'acétate de sodium.

On obtient ainsi finalement du polysaccharide C purifié correspondant sensiblement au produit de l'exemple 4.

## Exemple 14

La technique est celle de l'exemple 4 mais on procède au départ d'une culture de Neisseria meningitidis C-, on remplace la Celite 545 par de l'alumine neutre, le lavage avec la solution aqueuse de Cetavlon à 0,05 % par un lavage avec une solution aqueuse 0,1 N d'acétate de sodium contenant 0,1 % de Cetavlon, la composition finale de l'éluant est normale en chlorure de sodium dans de l'eau et on précipite par addition de 2 volumes d'éthanol.

On obtient ainsi finalement du polysaccharide C- purifié.

## Exemple 15

### Culture du microorganisme

Un échantillon d'une souche de Streptococcus

*pneumoniae* productrice de polysaccharide capsulaire de type 1 conservé à - 70° C dans le milieu Tryptic Soy Broth (un produit fabriqué et vendu par Difco Labs., Detroit, Michigan, U.S.A.) additionné de 10 % de glycérine est rapidement décongelé. L'organisme est recueilli à l'aide d'une anse en platine et transféré sur une boîte de Petri contenant 15 ml de milieu de culture constitué de 40 g de Blood agar base (un produit fabriqué et vendu par Difco Labs, Detroit, Michigan, U.S.A.) par litre d'eau, stérilisé à 121° C pendant 20 minutes et ensuite refroidi à 50° C et additionné de 5 % de sang de mouton frais et défibriné; le microorganisme est alors incubé à 37°C pendant 16 heures en atmosphère enrichie en $CO_2$(5 % p/v).

On recueille les bactéries produites et on procède à un second passage mais sur boîte de Roux contenant 100 ml de milieu synthétique suivant P.D. HOEPRICH (loc. cit.) solidifié par addition d'agar (20 g/l).

Après une période d'incubation de 6 à 9 heures en atmosphère anaérobe, l'organisme produit est récolté et mis en suspension dans 5 ml de solution aqueuse de chlorure de sodium (8,5 g par litre). La récolte sert à ensemencer un récipient de culture contenant 8 litres de milieu semi-synthétique liquide suivant P.D. HOEPRICH (loc. cit.). Le milieu est maintenu sans agitation ni aération pendant 16 heures à 37° C pour constituer l'inoculum de la culture de production.

Pour la culture de production, on utilise un fermenteur de 100 litres contenant 72 litres de milieu semi-synthétique suivant P.D. HOEPRICH (loc. cit.). Le milieu est ensemencé avec la culture précédente et l'incubation est maintenue sans aération mais sous faible agitation à 35,5° C ( ± 1), le pH étant continuellement ajusté à 7,2 à l'aide d'une solution de soude caustique à 25 %. Le développement de la culture est suivi régulièrement par échantillonnage et mesure de la densité optique du milieu à 620 nm. Lorsque cette densité optique atteint 0,15, on ajoute une solution d'un kg de dextrose dans 2 litres d'eau désionisée autoclavée pendant 20 minutes à 121° C.

La culture est arrêtée en début de phase stationnaire, ce qui correspond à une densité optique de 0,8.

## Extraction du polysaccharide

On transfère le milieu de fermentation (80 l) dans une cuve de stockage et on l'inactive par addition d'un litre de phénol à 90 %; le pH est ajusté à 7,2 avec une solution de soude caustique à 25 %. Après une nuit de repos à 5 - 10° C, on y dissout 1168 g de chlorure de sodium) ce qui correspond à une addition de 0,25 mole de chlorure de sodium par litre de milieu (dite concentration A).

Sous agitation, on ajoute ensuite 4 kg de support poreux inerte (Celite 545) et, à la suspension, on ajoute 800 ml (dit volume B) d'une solution aqueuse de Cetavlon (5 % p/v) préalablement conservée à une température de 25° C et on maintient l'agitation pendant 10 minutes après l'addition de Cetavlon.

Le milieu est alors filtré et le gâteau est lavé avec 4 l d'une solution aqueuse de chlorure de sodium de concentration A, additionnée de 40 ml d'une solution aqueuse de cetavlon (5 % p/v).

Le filtrat est concentré jusqu'à un volume de 5 litres par ultrafiltration dans un appareil AMICON DC 30 muni de cartouches d'un cut-off pour poids moléculaire de 100.000 daltons. Le rétentat est diafiltré d'abord avec 40 l dè solution de chlorure de sodium de concentration A et ensuite avec 40 l d'eau en maintenant le volume entre 10 et 5 litres. Finalement, on réduit le volume de la solution à environ 3 litres, par ultrafiltration et l'appareillage est rincé à l'eau pour obtenir environ 5 litres de solution de polysaccharide brut qui contient encore de la substance C.

Ce polysaccharide peut être isolé en dissolvant 40 g d'acétate de sodium trihydraté dans la solution et en ajoutant ensuite sous agitation 3,5 volumes d'éthanol, ce qui provoque la précipitation du polysaccharide type 1 qui peut être séparé par filtration.

## Purification du polysaccharide

Alternativement et, de préférence, on procède à une purification du polysaccharide en solution.

A cette fin, on dissout 34 g d'acétate de sodium trihydraté pour obtenir une solution saline (dite solution C) dont la concentration (dite concentration C) en acétate de sodium est 0,05 N, on ajuste le pH à 5,2 ( ± 0,1) avec de l'acide chlorhydrique N et on clarifie le milieu par filtrations successives sur 3 membranes dont la porosité est respectivement de 3, 1 et 0,45 micron.

Le polysaccharide est précipité sur 250 g d'agent filtrant (Celite 512) sous agitation et par addition de 500 ml (dit volume D) de solution aqueuse de Cetavlon à 5 % (p/v), l'agitation étant maintenue pendant 15 minutes après addition du Cetavlon.

On filtre alors le mélange sur une précouche de Celite 512.

Le précipité est lavé chromatographiquement avec 10 l d'une solution aqueuse d'acétate de sodium trihydraté (de concentration C) additionnée de 100 ml (dit volume E) de solution aqueuse de Cetavlon à 5 % (p/v).

Le gâteau est remis en suspension dans 2 litres l'une solution aqueuse 0,3 N (dite éluant F) d'acétate le sodium. Après 15 minutes sous agitation, la suspension est filtrée et le gâteau est lavé chromatographiquement avec 2 litres d'éluant F.

Les deux éluats sont filtrés sur un préfiltre de fibre de verre suivi de deux filtres à membrane présentant respectivement une porosité de 0,8 et de 0,45 micron. On ajoute de l'acétate de sodium trihydraté pour obtenir une concentration de 8 % et la solution est refiltrée sur un filtre à membrane de porosité 0,2 micron.

Le polysaccharide type 1 purifié est précipité de la solution par addition de 3 volumes d'éthanol.

Après décantation, le surnageant est séparé et le précipité est trituré dans de l'éthanol jusqu'à obtention d'une poudre homogène qui est filtrée et séchée sous pression réduite à température ordinaire. Le produit obtenu est du polysaccharide pneumococcique type 1 purifié.

**Exemple 16**

La technique est celle de l'exemple 15 mais, au départ, on utilise une souche de Streptococcus pneumoniae type 2; au stade d'extraction, la concentration A est 0,15 N, obtenue par addition de 702 g de chlorure de sodium et, au stade de purification, la solution C est 0,025 N, obtenue par addition de 17 g d'acétate de sodium trihydraté et l'éluant F est 0,15 N.

En fin d'opération, on obtient du polysaccharide pneumococcique type 2 purifié.

**Exemple 17**

La technique est celle de l'exemple 15 mais, au départ, on utilise une souche de Streptococcus pneumoniae type 3; au stade d'extraction, la concentration A est 0,3 N obtenue par addition de 1404 g de chlorure de sodium, la quantité de support poreux (Celite 545) est de 6 kg, le volume B est 1.600 ml, le filtrat est ultrafiltré jusqu'à 40 l, ensuite diafiltré successivement avec 160 l d'une solution 0,3 N de chlorure de sodium et avec 240 l d'une solution 0,125 N de chlorure de sodium et le volume du rétentat est ramené à 40 l (solution C); au stade de purification, on ajoute 6 kg de Celite 545 et, pour la suite des opérations, on utilise un facteur multiplicatif de 8 (dû au volume de la solution C, c'est-à-dire 40 l au lieu de 5 l) et l'éluant F est 0,6 N en acétate de sodium.

**Exemple 18**

La technique est celle de l'exemple 16 mais au départ, on utilise une souche de Streptococcus pneumoniae type 4 et on utilise 4 volumes d'éthanol pour la précipitation finale.

En fin d'opération, on obtient du polysaccharide pneumococcique type 4 purifié.

**Exemple 19**

La technique est celle de l'exemple 16 mais, au départ, on utilise une souche de Streptococcus pneumoniae type 5; au stade de purification, on ajuste le pH de la solution C à 5, l'éluant F est une solution aqueuse 0,15 N de chlorure de sodium et le polysaccharide est précipité par 3 volumes de n-propanol.

En fin d'opération, on obtient du polysaccharide type 5 purifié.

**Exemple 20**

La technique est celle de l'exemple 16 mais, au départ, on utilise une souche de Streptococcus pneumoniae type 6 A; au stade de purification, le volume D est 1000 ml, le volume E est 2000 ml et l'éluant F est 0,3 N en acétate de sodium.

En fin d'opération, on obtient du polysaccharide pneumococcique type 6 A purifié.

**Exemple 21**

La technique est celle de l'exemple 16 mais, au départ, on utilise une souche de Streptococcus pneumoniae type 6 B; au stade de purification, la concentration C est 0,05 N, obtenue par addition de 34 g d'acétate de sodium trihydraté, le volume D est 1000 ml, le volume E est 1000 ml et l'éluant F est 0,25 N.

En fin d'opération, on obtient du polysaccharide pneumococcique type 6 B purifié.

**Exemple 22**

La technique est celle de l'exemple 16 mais, au départ, on utilise une souche de Streptococcus pneumoniae type 8 et, au stade de purification, l'éluant F est 0,2 N.

En fin d'opération, on obtient du polysaccharide pneumococcique type 8 purifié.

**Exemple 23**

La technique est celle de l'exemple 16 mais, au départ, on utilise une souche de Streptococcus pneumoniae type 9 A et, au stade de purification, le volume E est 500 ml.

En fin d'opération, on obtient du polysaccharide pneumococcique type 9 A purifié.

**Exemple 24**

La technique est celle de l'exemple 16 mais, au départ, on utilise une souche de Streptococcus pneumoniae type 9 N.

En fin d'opération, on obtient du polysaccharide pneumococcique type 9 N purifié.

### Exemple 25

La technique est celle de l'exemple 16 mais, au départ, on utilise une souche de Streptococcus pneumoniae type 10 A et, au stade de purification, le volume D est 625 ml, le volume E est 600 ml et l'éluant F est 0,2 N.
En fin d'opération, on obtient du polysaccharide pneumococcique type 10 A purifié.

### Exemple 26

La technique est celle de l'exemple 16 mais, au départ, on utilise une souche de Streptococcus pneumoniae type 11 A et, au stade de purification, l'éluant F est 0,3 N.
En fin d'opération, on obtient du polysaccharide pneumococcique type 11 A purifié.

### Exemple 27

La technique est celle de l'exemple 16 mais, au départ, on utilise une souche de Streptococcus pneumoniae type 12 F.
En fin d'opération, on obtient du polysaccharide pneumococcique type 12 F purifié.

### Exemple 28

La technique est celle de l'exemple 18 mais, au départ, on utilise une souche de Streptococcus pneumoniae type 15 A.
En fin d'opération, on obtient du polysaccharide pneumococcique type 15 A purifié.

### Exemple 29

La technique est celle de l'exemple 16 mais, au départ, on utilise une souche de Streptococcus pneumoniae type 15 B et, au stade de purification, l'addition de la solution de Cetavlon (volume E) est remplacée par une addition de 10 % d'éthanol.
En fin d'opération, on obtient du polysaccharide pneumococcique type 15 B purifié.

### Exemple 30

La technique est celle de l'exemple 16 mais, au départ, on utilise une souche de Streptococcus pneumoniae type 15 C.
En fin d'opération, on obtient du polysaccharide pneumococcique type 15 C purifié.

### Exemple 31

La technique est celle de l'exemple 16 mais, au départ, on utilise une souche de Streptococcus pneumoniae type 15 F et, au stade de purification, on ajoute 10 % d'éthanol à la solution C, le volume D est 750 ml et le volume E est 1500 ml.
En fin d'opération, on obtient du polysaccharide pneumococcique type 15 F purifié.

### Exemple 32

La technique est celle de l'exemple 16 mais, au départ, on utilise une souche de Streptococcus pneumoniae type 17 F.
En fin d'opération, on obtient du polysaccharide pneumococcique type 17 F purifié.

### Exemple 33

La technique est celle de l'exemple 16 mais, au départ, on utilise une souche de Streptococcus pneumoniae type 18 C et, au stade de purification, la concentration C est 0,03 N obtenue par addition de 20,4 g d'acétate de sodium trihydraté.
En fin d'opération, on obtient du polysaccharide pneumococcique type 18 C purifié.

### Exemple 34

La technique est celle de l'exemple 26 mais, au départ, on utilise une souche de Streptococcus pneumoniae type 19 A.
En fin d'opération, on obtient du polysaccharide pneumococcique type 19 A purifié.

### Exemple 35

La technique est celle de l'exmple 26 mais, au départ, on utilise une souche de Streptococcus pneumoniae type 19 F.
En fin d'opération, on obtient du polysaccharide pneumococcique type 19 F purifié.

**Exemple 36**

La technique est celle de l'exemple 16 mais, au départ, on utilise une souche de Streptococcus pneumoniae type 20, au stade d'extraction, le volume B est 800 ml et, au stade de purification, on ajuste le pH de la solution C à 5 et le volume E est 500 ml.

En fin d'opération, on obtient du polysaccharide pneumococcique type 20 purifié.

**Exemple 37**

La technique est celle de l'exemple 16 mais, au départ, on utilise une souche de Streptococcus pneumoniae type 22 F et, au stade de purification, on ajuste le pH de la solution C à 5 et le volume E est 500 ml.

En fin d'opération, on obtient du polysaccharide pneumococcique type 22 F purifié.

**Exemple 38**

La technique est celle de l'exemple 16 mais, au départ, on utilise une souche de Streptococcus pneumoniae type 23 F et, au stade de purification, la solution C est une solution aqueuse 0,08 N de chlorure d'ammonium à pH 5,2, le volume D est 1500 ml, le volume E est 3000 ml et l'éluant F est une solution aqueuse 0,12 N de chlorure d'ammonium.

En fin d'opération, on obtient du polysaccharide pneumococcique type 23 F purifié.

**Exemple 39**

La technique est celle de l'exemple 23 mais, au départ, on utilise une souche de Streptococcus pneumoniae type 24 F.

En fin d'opération, on obtient du polysaccharide pneumococcique type 24 F purifié.

**Exemple 40**

La technique est celle de l'exemple 16 mais, au départ, on utilise une souche de Streptococcus pneumoniae type 25.

En fin d'opération, on obtient du polysaccharide pneumococcique type 25 purifié.

**Exemple 41**

La technique est celle de l'exemple 16 mais, au départ, on utilise une souche de Streptococcus pneumoniae type 33 B.

En fin d'opération, on obtient du polysaccharide pneumococcique type 33 B purifié.

**Exemple 42**

La technique est celle de l'exemple 16 mais, au départ, on utilise une souche de Streptococcus pneumoniae type 33 C.

En fin d'opération, on obtient du polysaccharide pneumococcique type 33 C purifié.

**Exemple 43**

La technique est celle de l'exemple 16 mais, au départ, on utilise une souche de Streptococcus pneumoniae type 35 A.

En fin d'opération, on obtient du polysaccharide pneumococcique type 35 A purifié.

**Exemple 44**

La technique est celle de l'exemple 16 mais, au départ, on utilise une souche de Streptococcus pneumoniae type 35 B et, au stade de purification, les 500 ml du volume D sont remplacés par 500 ml d'une solution de Cétavlon à 5 % (p/v) dans de l'éthanol, la solution de lavage C contient 10 % d'éthanol et l'éluant F est une solution aqueuse de 0,15 N de chlorure de sodium.

En fin d'opération, on obtient du polysaccharide pneumococcique type 35 B purifié.

**Exemple 45**

La technique est celle de l'exemple 26 mais, au départ, on utilise une souche de Streptococcus pneumoniae type 35 C.

En fin d'opération, on obtient du polysaccharide pneumococcique type 35 C purifié.

**Exemple 46**

La technique est celle de l'exemple 22 mais, au départ, on utilise une souche de Streptococcus pneumoniae type 35 F.

En fin d'opération, on obtient du polysaccharide pneumococcique type 35 F purifié.

**Exemple 47**

On procède, suivant la technique de l'exemple 16, mais au départ d'une souche de Streptococcus pneumoniae type 7 F jusqu'à l'obtention du polysaccharide brut precipité.

Ce précipité est redissous à raison de 4 g/l dans une solution tampon 0,005 N de trométamol/acide chlorhydrique à pH 7,1. La solution est clarifiée par centrifugation pendant 10 minutes à 3000 g.

Sous agitation, on ajoute, par litre de solution, 20 g de diéthylaminoéthylcellulose (DEAE cellulose) traitée par la même solution tampon, le pH est réajusté à 7,1 avec de l'acide chlorhydrique N et on maintient l'agitation pendant une heure après l'addition de la DEAE cellulose qui est alors séparée par filtration et lavée avec 240 ml de même solution tampon par litre de filtrat.

Le traitement à la DEAE cellulose est répété sur le filtrat.

Les filtrats réunis sont passés sur 2 membranes filtrantes présentant respectivement une porosité de 0,8 micron et de 0,45 micron. On ajoute de l'acétate de sodium trihydraté (pour que la concentration finale soit 8 % p/v) et la solution est passée sur membrane filtrante présentant une porosité de 0,2 micron.

On précipite le polysaccharide par addition de 3 volumes d'éthanol, sous agitation.

Par décantation suivie de filtration, on sépare le précipité de polysaccharide 7 F purifié qui est lavé à l'éthanol et finalement séché à température ordinaire sous pression réduite.

**Exemple 48**

On procède suivant la technique de l'exemple 47, mais au départ d'une souche de Streptococcus pneumoniae type 14 et, en fin d'opération, on obtient du polysaccharide type 14 purifié.

**Exemple 49**

On procède suivant la technique de l'exemple 47, mais au départ d'une souche de Streptococcus pneumoniae type 33 A et, en fin d'opération, on obtient du polysaccharide type 33 A purifié.

**Exemple 50**

On procède suivant la technique de l'exemple 47, mais au départ d'une souche de Streptococcus pneumoniae type 33 F et, en fin d'opération, on obtient du polysaccharide type 33 F purifié.

**Revendications**

1. Procédé pour la préparation d'un polysaccharide bactérien capsulaire antigénique purifié au départ d'un milieu de culture ou d'une solution le contenant, comprenant au moins un stade de précipitation par formation de complexe entre un dérivé polyionique du milieu - lequel dérivé polyionique est au moins ledit polysaccharide - et un sel d'ammonium quaternaire, caractérisé en ce que au moins une précipitation de complexe est effectuée sur un support poreux inerte constitué par de la cellulose, un sel alcalino-terreux substantiellement insoluble dans l'eau, de l'oxyde ou de l'hydroxyde d'aluminium, de l'oxyde de silicium, un silicate ou un aluminosilicate.

2. Procédé suivant la revendication 1 dans laquelle le sel d'ammonium quaternaire est le bromure de cétrimonium.

3. Procédé suivant la revendication 2, dans laquelle le support poreux inerte est une terre de diatomées.

4. Procédé pour la préparation d'un polysaccharide méningococcique antigénique purifié du groupe A, C, C⁻, W ou Y ou du polysaccharide antigénique de H. influenzae type b, au départ d'un milieu de culture correspondant ou d'une solution aqueuse contenant ledit polysaccharide, caractérisé en ce que:

(a)   on isole le polysaccharide par précipitation sous forme d'un complexe avec un sel d'ammonium quaternaire, en présence d'un support poreux inerte constitué par de la cellulose, un sel alcalino-terreux substantiellement insoluble dans l'eau, de l'oxyde ou de l'hydroxyde d'aluminium, de l'oxyde de silicium, un silicate ou un aluminosilicate;

(b)   on traite le précipité par une solution ionique aqueuse ou aqueuse/éthanol (jusqu'à 60 % v/v en éthanol) de telle sorte que la concentration saline finale soit comprise entre 0,1 et 0,6 N, et on récolte la solution;

(c)   on précipite le polysaccharide par addition subséquente d'éthanol;

(d)   on recueille le précipité qu'on dissout dans une solution ionique aqueuse;

(e)   on dialyse la solution et

(f)   on précipite le polysaccharide du rétentat par addition d'éthanol.

5. Procédé suivant la revendication 4 dans laquelle la solution ionique aqueuse au stade (d) est une solution 0,1 a 2 N de chlorure de calcium.

6. Procédé suivant l'une quelconque des revendications 4 et 5 dans laquelle le polysaccharide est le polysaccharide du groupe A ou du groupe C, caractérisé en ce que la concentration saline finale au stade (b) est 0,45 N en chlorure de sodium dans le mélange eau/éthanol 45/55.

7. Procédé suivant l'une quelconque des revendications 4 et 5 dans laquelle le polysaccharide est le polysaccharide du groupe C-, cacactérisé en ce que la concentration saline finale au stade (b) est 0,2 N en chlorure de sodium dans le mélange eau/éthanol 55/45.

8. Procédé suivant l'une quelconque des revendications 4 et 5 dans laquelle le polysaccharide est le polysaccharide du groupe W-135 ou Y, caractérisé en ce que la concentration saline finale au stade (b) est 0,11 N en chlorure de sodium dans le mélange eau/éthanol 48/52.

9. Procédé suivant l'une quelconque des revendications 4 et 5 dans laquelle le polysaccharide est le polysaccharide de H. influenzae type b, caractérisé en ce que la concentration saline finale au stade (b) est 0,6 N en chlorure de sodium dans de l'eau.

10. Procédé suivant l'une quelconque des revendications 4 à 9 dans laquelle le sel d'ammonium aliphatique à longue chaîne est le bromure de cétrimonium.

11. Procédé pour la préparation d'un polysaccharide pneumococcique anionique antigénique purifié au départ d'un milieu de culture ou d'une solution aqueuse le contenant, caractérisé en ce que:

(a) on élimine du milieu une partie des composants polyioniques indésirables, par précipitation sous forme de complexes avec un sel d'ammonium quaternaire en présence d'un support poreux inerte constitué par de la cellulose, un sel alcalino-terreux substantiellement insoluble dans l'eau, de l'oxyde ou de l'hydroxyde d'aluminium, de l'oxyde de silicium, un silicate ou un aluminosilicate et à une concentration ionique du milieu comprise entre 0,15 N et 1 N en sel;

(b) on recueille la solution de polysaccharide pneumococcique;

(c) on concentre le milieu et on ajuste sa concentration ionique à la valeur à laquelle se forme le complexe insoluble entre le polysaccharide et le sel d'ammonium quaternaire, en présence d'un support poreux inerte constitué par de la cellulose, un sel alcalino-terreux substantiellement insoluble dans l'eau, de l'oxyde ou de l'hydroxyde d'aluminium, de l'oxyde de silicium, un silicate ou un aluminosilicate, à pH 5 - 5,2;

(d) on recueille le polysaccharide complexé précipité sur ledit support inerte;

(e) on reprend le polysaccharide complexé dans une solution aqueuse de concentration ionique égale ou supérieure à 0,10 N en sel;

(f) on clarifie la solution et

(g) on précipite le polysaccharide purifié, par addition d'éthanol.

12. Procédé suivant la revendication 11 dans laquelle le sel d'ammonium aliphatique à longue chaîne est le bromure de cétrimonium.

13. Procédé suivant l'une quelconque des revendications 11 et 12 dans laquelle le polysaccharide est le polysaccharide pneumococcique type 2, 4, 9 A, 9 N, 12 F, 15 A, 15 B, 17 F, 18 C, 24 F, 25, 33 B, 33 C ou 35 A, caractérisé en ce que, au stade (c), la concentration ionique est 0,025 N en acétate de sodium et le pH est 5,2 et, au stade (e), la concentration ionique est 0,15 N en acétate de sodium dans de l'eau.

14. Procédé suivant l'une quelconque des revendications 11 et 12 dans laquelle le polysaccharide est le polysaccharide pneumococcique type 1, caractérisé en ce que, au stade (a), la concentration ionique est comprise entre 0,3 et 1 N; au stade (c), la concentration ionique est 0,05 N en acétate de sodium et le pH est 5 et, au stade (e), la concentration ionique est 0,3 N en acétate de sodium dans de l'eau.

15. Procédé suivant l'une quelconque des revendications 11 et 12 dans laquelle le polysaccharide est le polysaccharide pneumococcique type 3, caractérisé en ce que, au stade (a), la concentration ionique est comprise entre 0,3 et 1 N; au stade (c), la concentration ionique est 0,125 N en chlorure de sodium et le pH est 5,2 et, au stade (e), la concentration ionique est 0,6 N en acétate de sodium dans de l'eau.

16. Procédé suivant l'une quelconque des revendications 11 et 12 dans laquelle le polysaccharide est le polysaccharide pneumococcique 6 A, 11 A, 19 A, 19 F ou 35 C, caractérisé en ce que, au stade (c), la concentration ionique est 0,025 N en acétate de sodium et le pH est 5,2 et, au stade (e), la concentration ionique est 0,3 N en acétate de sodium dans de l'eau.

17. Procédé suivant l'une quelconque des revendications 11 et 12, dans laquelle le polysaccharide est le polysaccharide pneumococcique type à, 10 A ou 35 F, caractérisé en ce que, au stade (c), la concentration ionique est 0,025 N en acétate de sodium et le pH est 5,2 et, au stade (e), la concentration ionique est 0,2 N en acétate de sodium dans de l'eau.

18. Procédé suivant l'une quelconque des revendications 11 et 12 dans laquelle le polysaccharide est le polysaccharide pneumococcique type 15 F, caractérisé en ce que, au stade (c), la concentration ionique est 0,025 N en acétate de sodium dans un mélange eau/éthanol (90/10) et le pH est 5,2 et, au stade (e), la concentration ionique est 0,15 N en acétate de sodium dans de l'eau.

19. Procédé suivant l'une quelconque des revendications 11 et 12 dans laquelle le polysaccharide est le polysaccharide pneumococcique type 23 F, caractérisé en ce que, au stade (c), la concentration ionique est 0,08 N en chlorure d'ammonium et le pH est 5,2 et, au stade (e), la concentration ionique est 0,12 N en chlorure d'ammonium dans de l'eau.

20. Procédé suivant l'une quelconque des revendications 11 et 12 dans laquelle le polysaccharide est le polysaccharide pneumococcique type 5, caractérisé en ce que, au stade (c), la concentration ionique est 0,025 N en acétate de sodium et le pH est 5 et, au stade (e), la concentration ionique est 0,15 N en chlorure de sodium dans de l'eau.

21. Procédé suivant l'une quelconque des revendications 11 et 12 dans laquelle le polysaccharide est le polysaccharide pneumococcique type 6 B, caractérisé en ce que, au stade (c), la concentration ionique est 0,05 N en acétate de sodium et le pH est 5,2 et, au stade (e), la concentration ionique est 0,25 N en acétate de sodium dans de l'eau.

22. Procédé suivant l'une quelconque des revendications 11 et 12 dans laquelle le polysaccharide est le polysaccharide pneumococcique type 20 ou 22 F, caractérisé en ce que, au stade (c), la concentration ionique est 0,025 N en acétate de sodium et le pH est 5 et, au stade (e), la concentration ionique est 0,25 N en acétate de sodium dans de l'eau.

23. Procédé suivant l'une quelconque des revendications 11 et 12 dans laquelle le polysaccharide est le polysaccharide pneumococcique type 35 B, caractérisé en ce que, au stade (c), la concentration ionique est 0,025 N en acétate de sodium et le pH est 5,2 et, au stade (e), la concentration ionique est 0,15 N en chlorure de sodium dans de l'eau.

24. Procédé pour la préparation d'un polysaccharide pneumococcique non ionique antigénique purifié au départ d'un milieu de culture ou d'une solution aqueuse le contenant, caractérisé en ce que:

(a)    on élimine du milieu une partie des composants polyioniques indésirables, par précipitation sous forme de complexes avec un sel d'ammonium quaternaire en présence d'un support poreux inerte constitué par de la cellulose, un sel alcalino-terreux substantiellement insoluble dans l'eau, de l'oxyde ou de l'hydroxyde d'aluminium, de l'oxyde de silicium, un silicate ou un aluminosilicate;

(b)    on recueille la solution de polysaccharide pneumococcique;

(c)    on concentre le milieu et on précipite le polysaccharide, par addition de trois volumes d'éthanol;

(d)    on dissout le précipité dans une solution tampon cationique 0,005 N à pH 7 ( ± 0,1) et on traite la solution à la diéthylaminoéthyl cellulose et

(e)    on précipite le polysaccharide par addition d'éthanol.

25. Procédé suivant la revendication 24 dans laquelle le sel d'ammonium quaternaire est le bromure de cétrimonium.

26. Procédé suivant l'une quelconque des revendications 24 et 25 dans laquelle le polysaccharide est le polysaccharide pneumococcique type 7 F, 14, 33 A ou 33 F.

27. Procédé suivant l'une quelconque des revendications 1 à 26 dans laquelle on procède au départ d'un milieu de culture synthétique.

**Patentansprüche**

1. Verfahren zur Herstellung eines gereinigten bakteriellen antigenen Kapselpolysaccharids aus einem Kulturmedium oder einer es enthaltenden Lösung, umfassend mindestens eine Fällungsstufe durch Bildung eines Komplexes zwischen einem polyionischen Derivat des Mediums - dieses polyionische Derivat ist mindestens das genannte Polysaccharid - und einem quarternären Ammoniumsalz, dadurch gekennzeichnet, daß mindestens eine Fällung des Komplexes auf einem porösen inerten Träger vorgenommen wird, der aus Cellulose, einem im wesentlichen in Wasser unlöslichen Erdalkalisalz, Aluminiumoxid oder -hydroxid, Siliciumoxid, einem Silikat oder einem Aluminiumsilikat besteht.

2. Verfahren gemäß Anspruch 1, in welchem das quarternäre Ammoniumsalz Cetrimoniumbromid ist.

3. Verfahren gemäß Anspruch 2, in welchem der poröse inerte Träger eine Diatomeenerde ist.

4. Verfahren zur Herstellung eines gereinigten antigenen Meningokokken-Polysaccharids der Gruppe A, C, C⁻, W oder Y oder eines antigenischen H. Influenzae-Polysaccharid vom Typ b ausgehend von einem entsprechenden Kulturmedium oder einer wäßrigen, das genannte Polysaccharid enthaltenden Lösung, dadurch gekennzeichnet, daß man

(a)    das Polysaccharid durch Fällung unter Bildung eines Komplexes mit einem quarternären Ammoniumsalz in Gegenwart eines porösen inerten Trägers isoliert, der aus Cellulose, einem im wesentlichen in Wasser unlöslichen Erdalkalisalz, Aluminiumoxid oder -hydroxid, Siliziumoxid, einem Silikat oder einem Aluminiumsilikat besteht;

(b)    die Fällung derart mit einer wäßrigen ionischen oder wäßrig/ethanolischen (bis zu 60 % V/V Ethanol) Lösung behandelt, daß die Salzendkonzentration zwischen 0.1 und 0.6 N liegt und die Lösung gewinnt;

(c)    das Polysaccharid durch nachfolgenden Zusatz von Ethanol ausfällt;

(d)    die Ausfällung abtrennt, die man in einer wäßrigen ionischen Lösung auflöst;

(e)    die Lösung dialysiert und

(f)     das Polysaccharid des Retentats durch Zusatz von Ethanol ausfällt.

5. Verfahren gemäß Anspruch 4, in welchem die wäßrige ionische Lösung auf Stufe (d) eine 0.1 bis 2 N Calciumchloridlösung ist.

6. Verfahren gemäß einem der Ansprüche 4 und 5, in welchem das Polysaccharid das Polysaccharid der Gruppe A oder der Gruppe C ist, dadurch gekennzeichnet, daß die Salzendkonzentration in der Mischung Wasser/Ethanol 45/55 auf Stufe (b) 0.45 H an Natriumchlorid ist.

7. Verfahren gemäß einem der Ansprüche 4 und 5, in welchem das Polysaccharid das Polysaccharid der Gruppe C ist, dadurch gekennzeichnet, daß die Salzendkonzentration in Stufe (b) 0.2 N an Natriumchlorid in der Mischung Wasser/Ethanol 55/45 ist.

8. Verfahren gemäß einem der Ansprüche 4 und 5, in welchem das Polysaccharid das Polysaccharid der Gruppe W-135 oder Y ist, dadurch gekennzeichnet, daß die Salzendkonzentration in der Stufe (b) 0.11 N an Natriumchlorid in der Mischung Wasser/Ethanol 48/52 ist.

9. Verfahren gemäß einem der Ansprüche 4 und 5, in welchem das Polysaccharid das Polysaccharid von H. influenzae Typ b ist, dadurch gekennzeichnet, daß die Salzendkonzentration in Stufe (b) 0.6 N an Natriumchlorid in Wasser ist.

10. Verfahren gemäß einem der Ansprüche 4 bis 9, in welchem das langkettige aliphatische Ammoniumsalz Cetrimoniumbromid ist.

11. Verfahren zur Herstellung eines gereinigten antigenen anionischen Pneumokokken-Polysaccharids ausgehend von einem Kulturmedium oder einer wäßrigen es enthaltenden Lösung, dadurch gekennzeichnet, daß man

(a)     aus dem Medium einen Teil der unerwünschten polyionischen Bestandteile durch Fällung unter Bildung von Komplexen mit einem quarternären Ammoniumsalz in Gegenwart eines porösen inerten Trägers, der aus Cellulose, einem im wesentlichen in Wasser unlöslichen Erdalkalisalz, Aluminiumoxid oder -hydroxid, Siliziumoxid, einem Silikat oder einem Aluminiumsilikat besteht, und bei einer Ionenkonzentration des Mediums zwischen 0.15 N und 1 N an Salz eliminiert;

(b)     die Lösung von Pneumokokken-Polysaccharid gewinnt;

(c)     das Medium einengt und seine Ionenkonzentration auf einen Wert anpaßt, bei welchem sich der unlösliche Komplex zwischen dem Polysaccharid und dem quarternären Ammoniumsalz in Gegenwart eines porösen inerten Trägers bei einem pH von 5 - 5.2 bildet, der aus Cellulose, einem im wesentlichen in Wasser unlöslichen Erdalkalisalz, Aluminiumoxid oder -hydroxid, Siliziumoxid, einem Silikat oder einem Aluminiumsilikat besteht;

(d)     den auf dem genannten inerten Träger ausgefällten Polysaccharid-Komplex gewinnt;

(e)     den Polysaccharid-Komplex in einer wäßrigen Lösung mit einer Ionenkonzentration, die gleich oder größer als 0.10 N an Salz ist, aufnimmt;

(f)     die Lösung klärt und

(g)     das gereinigte Polysaccharid durch Zusatz von Ethanol ausfällt.

12. Verfahren gemäß Anspruch 11, in welchem das quarternäre Ammoniumsalz Cetrimoniumbromid ist.

13. Verfahren gemäß einem der Ansprüche 11 und 12, in welchem das Polysaccharid das Pneumokokken-Polysaccharid vom Typ 2, 4, 9 A, 9 N, 12 F, 15 A, 15 B, 17 F, 18 C, 24 F, 25, 33 B, 33 C oder 35 A ist, dadurch gekennzeichnet, daß in Stufe (c) die Ionenkonzentration 0.025 N an Natriumacetat ist und der pH 5.2 ist und in Stufe (e) die Ionenkonzentration 0.15 N an Natriumacetat in Wasser ist.

14. Verfahren gemäß einem der Ansprüche 11 und 12, in welchem das Polysaccharid das Pneumokokken-Polysaccharid vom Typ 1 ist, dadurch gekennzeichnet, daß in Stufe (a) die Ionenkonzentration zwischen 0.3 und 1 N liegt; in Stufe (c) die Ionenkonzentration 0.05 N an Natriumacetat ist und der pH 5 ist und in Stufe (e) die Ionenkonzentration 0.3 N an Natriumacetat in Wasser ist.

15. Verfahren gemäß einem der Ansprüche 11 und 12, in welchem das Polysaccharid das Pneumokokken-Polysaccharid vom Typ 3 ist, dadurch gekennzeichnet, daß in Stufe (a) die Ionenkonzentration zwischen 0.3 und 1 N liegt; in Stufe (c) die Ionenkonzentration 0.125 N an Natriumchlorid ist und der pH 5.2 ist und in Stufe (e) die Ionenkonzentration 0.6 N an Natriumacetat in Wasser ist.

16. Verfahren gemäß einem der Ansprüche 11 und 12, in welchem das Polysaccharid das Pneumokokken-Polysaccharid 6 A, 11 A, 19 A, 19 F oder 35 C ist, dadurch gekennzeichnet, daß in Stufe (c) die Ionenkonzentration 0.025 N an Natriumacetat ist und der pH 5.2 ist und in Stufe (e) die Ionenkonzentration 0.3 N an Natriumacetat in Wasser ist.

17. Verfahren gemäß einem der Ansprüche 11 und 12, in welchem das Polysaccharid das Pneumokokken-Polysaccharid vom Typ 8, 10 A oder 35 F ist, dadurch gekennzeichnet, daß in Stufe (c) die Ionenkonzentration 0.025 N an Natriumacetat ist und der pH 5.2 ist und in Stufe (e) die Ionenkonzentration 0.2 N an Natriumacetat in Wasser ist.

18. Verfahren gemäß einem der Ansprüche 11 und 12, in welchem das Polysaccharid das Pneumokokken-Polysaccharid vom Typ 15 F ist, dadurch gekennzeichnet, daß in Stufe (c) die Ionenkonzentration 0.025 N an Natriumacetat in einer Mischung von Wasser/Ethanol (90/10) ist und der pH 5.2 ist und in Stufe (e) die lonenkonzentration 0.15 N an Natriumacetat in Wasser ist.

19. Verfahren gemäß einem der Ansprüche 11 und 12, in welchem das Polysaccharid das Pneumokokken-Polysaccharid vom Typ 23 F ist, dadurch gekennzeichnet, daß in Stufe (c) die lonenkonzentration 0.08 N an Ammoniumchlorid ist und der pH 5.2 ist und in Stufe (e) die lonenkonzentration 0.12 N an Ammoniumchlorid in Wasser ist.

20. Verfahren gemäß einem der Ansprüche 11 und 12, in welchem das Polysaccharid das Pneumokokken-Polysaccharid vom Typ 5 ist, dadurch gekennzeichnet, daß in Stufe (c) die lonenkonzentration 0.025 N an Natriumacetat ist und der pH 5 ist und in Stufe (e) die lonenkonzentration 0.15 N an Natriumchlorid in Wasser ist.

21. Verfahren gemäß einem der Ansprüche 11 und 12, in welchem das Polysaccharid das Pneumokokken-Polysaccharid vom Typ 6 B ist, dadurch gekennzeichnet, daß in Stufe (c) die lonenkonzentration 0.05 N an Natriumacetat ist und der pH 5.2 ist und in Stufe (e) die lonenkonzentration 0.25 N an Natriumacetat in Wasser ist.

22. Verfahren gemäß einem der Ansprüche 11 und 12, in welchem das Polysaccharid das Pneumokokken-Polysaccharid vom Typ 20 oder 22 F ist, dadurch gekennzeichnet, daß in Stufe (c) die lonenkonzentration 0.025 N an Natriumacetat ist und der pH 5 ist und in Stufe (e) die lonenkonzentration 0.25 N an Natriumacetat in Wasser ist.

23. Verfahren gemäß einem der Ansprüche 11 und 12, in welchem das Polysaccharid das Pneumokokken-Polysaccharid vom Typ 35 B ist, dadurch gekennzeichnet, daß in Stufe (c) die lonenkonzentration 0.025 N an Natriumacetat ist und der pH 5.2 ist und in Stufe (e) die lonenkonzentration 0.15 N an Natriumchlorid in Wasser ist.

24. Verfahren zur Herstellung eines gereinigten antigenen nicht-ionischen Pneumokokken-Polysaccharids ausgehend von einem Kulturmedium oder einer wäßrigen es enthaltenden Lösung, dadurch gekennzeichnet, daß man

(a)	aus dem Medium einen Teil der unerwünschten polyionischen Bestandteile durch Fällung unter Bildung von Komplexen mit einem quarternären Ammoniumsalz in Gegenwart eines porösen inerten Trägers eliminiert, der aus Cellulose, einem im wesentlichen in Wasser unlöslichen Erdalkalisalz, Aluminiumoxid oder -hydroxid, Siliziumoxid, einem Silikat oder einem Aluminiumsilikat besteht;

(b)	die Lösung von Pneumokokken-Polysaccharid gewinnt;

(c)	das Medium einengt und das Polysaccharid durch Zusatz von drei Volumina Ethanol ausfällt;

(d)	die Fällung in einer 0.0005 N kationischen Pufferlösung von pH 7 (± 0.1) löst und die Lösung mit Diethylaminoethylcellulose behandelt und

(e)	das Polysaccharid durch Zusatz von Ethanol ausfällt.

25. Verfahren gemäß Anspruch 24, in welchem das quaternäre Ammoniumsalz Cetrimoniumbromid ist.

26. Verfahren gemäß einem der Ansprüche 24 und 25, in welchem das Polysaccharid das Pneumokokken-Polysaccharid vom Typ 7 F, 14, 33 A oder 33 F ist.

27. Verfahren gemäß einem der Ansprüche 1 bis 26, in welchem man von Anfang an von einem synthetischen Kulturmedium ausgeht.

**Claims**

1. Process for the preparation of an antigenic capsular bacterial polysaccharide from a culture medium or a solution containing it comprising at least one precipitation step by formation of a complex between a polyionic derivative of the medium - which polyionic derivative is at least said polysaccharide - and a quaternary ammonium salt, characterized in that at least one precipitation of the complex is performed on an inert porous support constituted of cellulose, an alkaline-earth metal salt substantially insoluble in water, aluminium oxide or hydroxide, silicon oxide, a silicate or an aluminosilicate.

2. Process according to claim 1 wherein the quaternary ammonium salt is cetrimonium bromide.

3. Process according to claim 2 wherein the inert porous support is a diatomaceous earth.

4. Process for the preparation of a purified antigenic meningococcic polysaccharide of group A, C, C-, W or Y or the antigenic polysacchariede of H. influenza type b from a corresponding culture medium or from an aqueous solution containing said polysaccharide characterized in that:

(a)	the polysaccharide is isolated by precipitation in the form of a complex with a quaternary ammonium salt in the presence of an inert porous support constituted of cellulose, an alkaline-earth metal salt substantially insoluble in water, aluminium oxide or hydroxide, silicium oxide, a silicate or aluminosilicate;

(b)	the precipitate is treated with an ionic water or water/ethanol solution (up to 60 % ethanol v/v) in such a manner that the final salt solution is comprised between 0.1 and 0.6 N and the solution is harvested;

(c)	the polysaccharide is precipitated by subsequent addition of ethanol;

(d)	the precipitate is collected and dissolved in an ionic aqueous solution;

(e)	the solution is dialysed and

(f)	the polysaccharide is precipitated from the retentate by addition of ethanol.

5. Process according to claim 4 wherein the ionic aqueous solution of step (d) is a 0.1 to 0.2 N calcium chloride solution.

6. Process according to any of claims 4 and 5 wherein the polysaccharide is a polysaccharide group A or group C, characterized in that the final salt solution in step (b) is 0.45 N sodium chloride in the 45/55 water/ethanol mixture.

7. Process according to any of claims 4 and 5 wherein the polysaccharide is polysaccharide group C, characterized in that the final salt concentration in step (b) is 0.2 N sodium chloride in the 55/45 water/ethanol mixture.

8. Process according to any of claims 4 and 5 wherein the polysaccharide is a polysaccharide group W-135 or Y, characterized in that the final salt concentration is step (b) is 0.11 N sodium chloride in the 48/52 water/ethanol mixture.

9. Process according to any of claims 4 and 5 wherein the polysaccharide is the polysaccharide H. influenza type b, characterized in that the final salt concentration is step (b) is 0.6 N sodium chloride in water.

10. Process according to any of claims 4 to 9 wherein the quaternary ammonium salt is cetrimonium bromide.

11. Process for the preparation of a purified antigenic anionic pneumococcal polysaccharide from a culture medium or an aqueous solution containing it, characterized in that:

(a)   a fraction of the undesirable polyionic components of the medium is eliminated by precipitation in the form of complexes with a quaternary ammonium salt in the presence of an inert porous support constituted of cellulose, an alkaline-earth metal salt substantially insoluble in water, aluminium oxide or hydroxide, silicon oxide, a silicate or aluminosilicate at an ionic concentration of the medium comprised between 0.15 N and 1 N in salt;

(b)   the solution of pneumococcal polysaccharide is collected;

(c)   the medium is concentrated and its ionic concentration is adjusted to the value at which the insoluble complex is formed between the polysaccharide and the quaternary ammonium salt, in the presence of an inert porous support constituted of cellulose, an alkaline-earth metal salt substantially insoluble in water, aluminium oxide or hydroxide, silicon oxide, a silicate or aluminosilicate at pH 5 - 5.2;

(d)   the complexed polysaccharide precipitated on said inert support is harvested;

(e)   the complexed polysaccharide is taken up in an aqueous solution of ionic concentration equal to or higher than 0.10 N in salt;

(f)   the solution is clarified and

(g)   the purified polysaccharide is precipitated by addition of ethanol.

12. Process according to claim 11 wherein the quaternary ammonium salt is cetrimonium bromide.

13. Process according to any of claims 11 and 12 wherein the polysaccharide is a pneumococcal polysaccharide of type 2, 4, 9 A, 9 N, 12 F, 15 A, 15 B, 17 F, 18 C, 24 F, 25, 33 B, 33 C or 35 A characterized in that, in step (c), the ionic concentration is 0.025 N sodium acetate and the pH is 5.2 and, in step (e), the ionic concentration is 0.15 N sodium acetate in water.

14. Process according to any of claims 11 and 12 wherein the polysaccharide is pneumococcal polysaccharide type 1, characterized in that, in step (a), the ionic concentration is comprised between 0.3 and 1 N; in step (c), the ionic concentration is 0.05 N sodium acetate and the pH is 5 and in step (e), the ionic concentration is 0.3 N sodium acetate in water.

15. Process according to any of claims 11 and 12 wherein the polysaccharide is pneumococcal polysaccharide type 3, characterized in that, in step (a), the ionic concentration is comprised between 0.3 and 1 N; in step (c), the ionic concentration is 0.125 N sodium chloride and the pH is 5.2 and, in step (e), the ionic concentration is 0.6 N sodium acetate in water.

16. Process according to any of claims 11 and 12 wherein the polysaccharide is pneumococcal polysaccharide 6 A, 11 A, 19 A, 19 F or 35 F, characterized in that, in step (c), the ionic concentration is 0.025 N sodium acetate and the pH is 5.2 and, in step (e), the ionic concentration is 0.3 N sodium acetate in water.

17. Process according to any of claims 11 and 12, wherein the polysaccharide is pneumococcal polysaccharide type 8, 10 A or 35 F, characterized in that, in step (c), the ionic concentration is 0.025 N sodium acetate ind the pH is 5.2 and, in step (e), the ionic concentration is 0.2 N sodium acetate in water.

18. Process according to any of claims 11 and 12 wherein the polysaccharide is pneumococcal polysaccharide type 15 F, characterized in that, in step (c), the ionic concentration is 0.025 N sodium acetate in water/ethanol mixture (90/10) and the pH is 5.2 and, in step (e), the ionic concentration is 0.15 N sodium acetate in water.

19. Process according to any of claims 11 and 12 wherein the polysaccharide is pneumococcal polysaccharide type 23 F, characterized in that the ionic concentration is 0.08 N ammonium chloride and the pH is 5.2 and, in step (e), the ionic concentration is 0.12 N ammonium chloride in water.

20. Process according to any of claims 11 and 12 wherein the polysaccharide is pneumococcal polysaccharide type 5, characterized in that, in step (c), the ionic concentration is 0.025 N sodium acetate and the pH is 5 and, in step (e), the ionic concentration is 0.15 N sodium chloride in water.

21. Process according to any of claims 11 and 12 wherein the polysaccharide is pneumococcal polysaccharide type 6 B, characterized in that, in step (c), the ionic concentration is 0.05 N sodium acetate and the pH is 5.2 and, in step (e), the ionic concentration is 0.25 N sodium acetate in water.

22. Process according to any of claims 11 and 12 wherein the polysaccharide is pneumococcal polysaccharide type 20 or 22 F, characterized in that, in step (c), the ionic concentration is 0.025 N sodium acetate and the pH is 5 and, in step (e), the ionic concentration is 0.25 N sodium acetate in water.

22. Process according to any of claims 11 and 12 wherein the polysaccharide is pneumococcal polysaccharide type 35 B, characterized in that, in step (c), the ionic concentration is 0.025 N sodium acetate and the pH is 5.2 and, in step (e), the ionic concentration is 0.15 N sodium chloride in water.

24. Process for the preparation of a purified antigenic non ionic pneumococcal polysaccharide from a culture medium on an aqueous solution containing it, characterized in that:

(a) a fraction of the undesirable polyionic components is eliminated by precipitation under the form of complexes with a quaternary ammonium salt in the presence of an inert porous support constituted of cellulose, an alkaline-earth metal salt substantially insoluble in water, aluminium oxide or hydroxide, silicon oxide, a silicate or alumino silicate;

(b) the solution of pneumococcal polysaccharide is collected;

(c) the medium is concentrated and the polysaccharide is precipitated by addition of three volumes of ethanol;

(d) the precipitate is dissolved in a cationic buffer solution 0.005 N pH 7 ($\pm$ 0.1) and the solution is treated with diethylaminoethyl cellulose and

(e) the polysaccharide is precipitated by addition of ethanol.

25. Process according to claim 24 wherein the quaternary ammonium salt is cetrimonium bromide.

26. Process according to any of claims 24 and 25 wherein the polysaccharide is pneumococcal polysaccharide type 7 F, 14, 33 A or 33 F.

27. Process according to any of claims 1 to 26 wherein the starting medium is a synthetic culture medium.